# FASCICULE DE BREVET EUROPEEN

(11) **EP 1 610 715 B1**
(45) Date de publication et mention de la délivrance du brevet: **28.11.2018**
(21) Numéro de dépôt: 04742370.2
(22) Date de dépôt: 26.03.2004
(51) Int. Cl.: A61B 17/06, A61B 17/34, A61B 17/00, A61F 2/00

(54) **IMPLANT POUR LE TRAITEMENT DE LA CYSTOCELE ET DISPOSITIF POUR LA MISE EN PLACE DE CET IMPLANT**
IMPLANTAT ZUR BEHANDLUNG DER CYSTOCELE UND VORRICHTUNG ZUM EINSETZEN JENES IMPLANTATS.
IMPLANT FOR THE TREATMENT OF A CYSTOCELE AND DEVICE FOR PLACEMENT OF SAID IMPLANT

(30) Priorité: 28.03.2003 FR 0303895
(43) Date de publication de la demande: 04.01.2006
(73) Titulaire: Analytic Biosurgical Solutions - ABISS, 42000 Saint-Étienne (FR)
(72) Inventeur: DELORME, Emmanuel, F-71100 Chalon sur Saone (FR); EGLIN, Georges, F-34500 Beziers (FR); BERAUD, Jean-Marc, F-42100 St Etienne (FR)
(74) Mandataire: Le Cacheux, Samuel L.R.
(86) Numéro de dépôt international: PCT/FR2004/000766
(87) Numéro de publication internationale: WO 2004/091443

(56) Documents cités:
- EP-A- 0 774 240
- EP-A1- 0 608 684
- WO-A-01/06951
- WO-A-02/38079
- WO-A-02/058563
- FR-A- 2 785 521

## Description

La présente invention concerne le domaine technique du traitement de la cystocèle notamment chez la femme âgée.

Les phénomènes de cystocèle résultent, généralement, d'un relâchement des tissus de suspension des organes urinaires et génitaux, entraînant des troubles qui nécessitent une intervention chirurgicale.

Ainsi, il a été proposé des tentatives de reconstruction du système naturel de suspension des organes, affectés par le relâchement, en mettant en oeuvre des sutures non résorbables ou des bandelettes de renfort. Cependant, ces techniques n'ont pas toujours donné satisfaction, notamment en raison du recours à une intervention chirurgicale lourde, entraînant une dissection de régions anatomiques non concernées par la réparation chirurgicale, pour la réalisation des points de sutures non résorbables.

Afin de tenter de remédier à ces inconvénients, une demande de brevet FR 2 785 521 a proposé de mettre en oeuvre un implant comprenant un corps de support, à partir duquel s'étendent deux cordons de suspension, pourvus, à leurs extrémités, de pièces d'ancrage destinées à faire l'objet de sutures sur des zones réputées pour être anatomiquement stables. Cet implant est alors mis en place par voie laparoscopique permettant d'alléger la procédure chirurgicale
Cependant, il est apparu qu'un tel implant n'est pas en mesure d'assurer une suspension efficace, en raison, notamment, des contraintes appliquées aux zones réputées pour être anatomiquement stables. De plus, ce type d'implants ne présente pas, en condition d'utilisation, une grande stabilité spatiale. Le document WO 02/38079 décrit un implant pour le traitement de la cystocèle et destiné à maintenir la vessie d'une femme, présentant une structure mince et souple, comprenant un corps de support et deux bretelles de suspension antérieures de part et d'autre d'un plan sagittal et de deux bretelles de suspension postérieures de part et d'autre du plan sagittal.

Ainsi, il est apparu le besoin de disposer, d'une part, d'un implant offrant une meilleure stabilité d'implantation-et, d'autre part, d'une technique qui permette d'obtenir une stabilité optimale, tout en réduisant au maximum le traumatisme subi par la patiente.

Ainsi, afin d'atteindre ces objectifs, l'invention concerne un implant selon la revendication 1.

Selon une caractéristique essentielle de l'invention, l'implant comprend, en outre, des bretelles de suspension, dites moyennes, qui s'étendent à partir du corps de support de part et d'autre du plan sagittal et entre les bretelles antérieures et postérieures.

La mise en oeuvre de six bretelles de suspension permet alors d'assurer une meilleure répartition des efforts subis par le corps de support sur les points d'ancrage anatomiques des bretelles, tout en garantissant la meilleure orientation spatiale du corps du support implanté chez la patiente.

Selon une caractéristique préférée mais non strictement nécessaire de l'invention, les bretelles de suspension antérieures sont destinées chacune à être engagée dans l'un des deux trous ischio-pubien encore appelés obturateurs ou obturés et présentent, à cet effet, une longueur supérieure à 100 mm et, de préférence, supérieure à 120 mm.

Selon une autre caractéristique de l'invention, les axes longitudinaux des bretelles antérieures forment, entre eux, un angle α supérieur à 45° et, de manière préférée mais non strictement nécessaire, un angle α compris entre 100° et 180° et, de préférence, entre 115° et 170°. Il est à noter que, selon une forme de réalisation préférée, le plan sagittal constitue un axe de symétrie de l'implant et donc bissecteur de l'angle α.

Selon une autre caractéristique de l'invention, les axes longitudinaux des bretelles postérieures forment, entre eux, un angle β non nul. Ainsi, il doit être considéré que les bretelles postérieures ne sont pas parallèles entre elles. De manière préférée mais non strictement nécessaire, l'angle β est supérieur à 10° pour être, de préférence, compris entre 10° et 75° ou, encore, 100° et 180°, selon la pathologie devant être traitée.

De manière préférée, les bretelles postérieures présentent une longueur supérieure à 100 mm et, de préférence, à 120 mm.

Selon une autre caractéristique de l'invention, les bretelles de suspension moyennes présentent, également, une longueur supérieure à 100 mm et, de préférence, supérieure à 120 mm.

Selon encore une autre caractéristique de l'invention, préférée mais non strictement nécessaire, le corps de support présente une forme générale sensiblement rectangulaire. De manière préférée mais non strictement nécessaire, le corps de support présente alors une longueur comprise entre 60 mm et 90 mm et une largeur comprise entre 40 mm et 60 mm.

Selon une forme préférée de réalisation de l'invention, les bretelles antérieures s'étendent sensiblement à partir des coins antérieurs du corps de support et les bretelles postérieures s'étendent également à partir des coins postérieurs du corps de support.

L'invention concerne également une technique opératoire pour la mise en place de l'implant ci-dessus et le traitement de la cystocèle.

Selon l'invention, la caractéristique essentielle de cette technique consiste à venir placer les bretelles de suspension antérieures chacune dans l'un des deux trous ischio-pubien, encore appelés trous obturateurs ou obturés.

Les bretelles de suspension moyennes sont quant à elles placées chacune, soit dans la région translévatorienne moyenne, soit dans un trou obturé dans sa zone inféropostérieure.

Tandis que les bretelles postérieures sont soit trans-fixées dans les ligaments utérosacrés soit placées au travers des ligaments utérosacrés puis dans la région transglutéale, de préférence mais non exclusivement, au travers des ligaments sacrosciatiques.

Il est à noter que selon cette technique le maintien est assuré principalement par friction entre l'implant et les tissus traversés et plus particulièrement par friction entre les bras de suspension et les tissus musculaires traversés.

De manière préférée, cette technique est mise en oeuvre dans le cadre d'une chirurgie, dite mini-invasive, utilisant des voies, d'abord proches des organes à traiter, les plus réduites possibles, de manière à assurer une réduction des temps opératoires et des pertes sanguines. Il est ainsi possible obtenir des suites opératoires courtes, avec un inconfort minime pour la patiente. De plus, la simplicité de cette technique permet sa reproduction aisée et réduit au maximum l'apprentissage nécessaire aux chirurgiens pour sa maîtrise.

L'invention concerne, également, un dispositif d'introduction qui peut être utilisé, sans que cela soit absolument nécessaire au sens de l'invention, pour la mise en place d'un implant, tel que décrit précédemment.

Selon l'invention, ce dispositif d'introduction comprend un introducteur qui présente une structure souple et une forme analogue à celle de l'implant et qui comprend :
▪ un corps creux définissant une cavité de réception du corps de l'implant,
▪ des branches tubulaires s'étendant à partir du corps creux et définissant chacune une cavité de réception d'une bretelle de suspension de l'implant,
▪ des moyens de traction s'étendant à partir de l'extrémité de chacune branche de l'introducteur,
▪ et des moyens de découpe du corps creux au moins de l'introducteur.

Selon une caractéristique de l'invention, les moyens de traction comprennent, pour chaque branche tubulaire, une aiguille semi-rigide.

Selon l'invention, les moyens de découpe peuvent être réalisés de toute façon appropriée, telle que, par exemple, sous la forme d'une ligne de moindre résistance susceptible de se déchirer lorsqu'une traction est effectuée au niveau de deux branches tubulaires opposées du dispositif introducteur.

Selon une caractéristique préférée mais non strictement nécessaire de l'invention, les moyens de découpe comprennent au moins une ouverture pour le passage d'un outil de coupe. De manière préférée, le corps de l'introducteur comprend une série d'ouvertures se situant d'une part à proximité d'une ligne correspondant au plan sagittal de l'implant et d'autre part entre les branches tubulaires.

Selon une autre caractéristique de l'invention, le dispositif d'introduction comprend un implant conforme à l'invention, disposé dans la cavité du corps creux et des branches tubulaires de l'introducteur. De manière préférée mais non strictement nécessaire, l'implant est libre à l'intérieur de l'introducteur.

Afin de faciliter le travail du chirurgien, selon une autre caractéristique de l'invention, le dispositif d'introduction comprend également au moins un ancillaire qui comprend un guide perforateur allongé ou trocart dont une extrémité est destinée à être introduite dans le corps de la patiente et dont l'autre extrémité est pourvue d'une poignée.

Selon une caractéristique de l'invention, le guide perforateur présente une forme arquée dans un plan. De manière préférée mais non strictement nécessaire, la partie arquée du perforateur s'étend sur un secteur angulaire supérieur à 140° et, de préférence mais non nécessairement, inférieur à 180° et, de préférence, compris entre 150° et 170°. De manière préférée, la partie arquée du guide perforateur présente alors un rayon de courbure compris entre 30 mm et 60 mm et, de préférence, pour la partie du guide perforateur s'étendant entre la poignée et l'extrémité destinée à être introduite dans le corps du patient, compris entre 40 mm et 50 mm, la partie extrême du perforateur présentant alors un rayon de courbure variable.

Selon une autre caractéristique de l'invention, le guide perforateur présente, au niveau de son extrémité opposée à la poignée ou distale, une forme hélicoïdale. De manière préférée, le guide perforateur présente alors la forme d'une portion de spire hélicoïdale s'étendant sur un angle compris entre 180° et 360° et, de préférence, compris entre 255° et 270°. De même, de manière préférée, la spire du guide perforateur présente un rayon de courbure compris entre 20 mm et 40 mm, avec un pas compris entre 15 mm et 25 mm.

Selon encore une autre caractéristique de l'invention, afin de réduire les traumatismes subis par le corps de la patiente lors de l'introduction de l'implant, le dispositif d'introduction comprend en outre une chemise tubulaire de forme complémentaire à celle du guide perforateur. Cette chemise tubulaire est alors destinée à être engagée sur le guide perforateur et à rester dans le corps de la patiente après retrait du guide perforateur pour définir un tunnel pour le passage des moyens de traction de l'introducteur. La chemise tubulaire est ensuite retirée, après passage des moyens de traction lors du retrait de l'introducteur.

Selon l'invention, la chemise tubulaire peut être réalisée en tout matériau souple biocompatible, tel que, par exemple mais non exclusivement, du PVC.

Diverses autres caractéristiques de l'invention ressortent de la description ci-dessous effectuée en référence aux dessins annexés qui illustrent différentes formes de réalisation d'un implant selon l'invention, ainsi que de dispositifs introducteurs permettant de faciliter la mise en place dudit implant.

Par ailleurs, il doit être noté que les différentes caractéristiques de l'invention, décrites précédemment et ci-après, peuvent être combinées ensemble selon différentes variantes, en fonction de la pathologie à traiter.
La **fig. 1** est une élévation, en vue à plat, d'un implant selon l'invention, plus particulièrement destiné au traitement de la cystocèle.
La **fig. 2** est une élévation, partiellement arrachée, d'un guide perforateur pouvant être utilisé pour la mise en place de l'implant selon l'invention et présentant une forme arquée.
La **fig. 3** est une élévation d'une autre forme de réalisation d'un guide perforateur selon l'invention, présentant une extrémité d'introduction de forme hélicoïdale.
La **fig. 4** est une vue de gauche du guide perforateur selon la **fig. 3****.**
La **fig. 5** est une vue de dessous du perforateur illustré à la **fig. 3****.**
La **fig. 6** est une vue d'un introducteur selon l'invention, permettant la mise en place de l'implant illustré à la **fig. 1****.**
Les **fig. 7** à **10** sont des vues, analogues aux **fig. 2** à **3****,** montrant des variantes de réalisation de guides perforateurs pour la mise en place d'un implant conforme à l'invention.

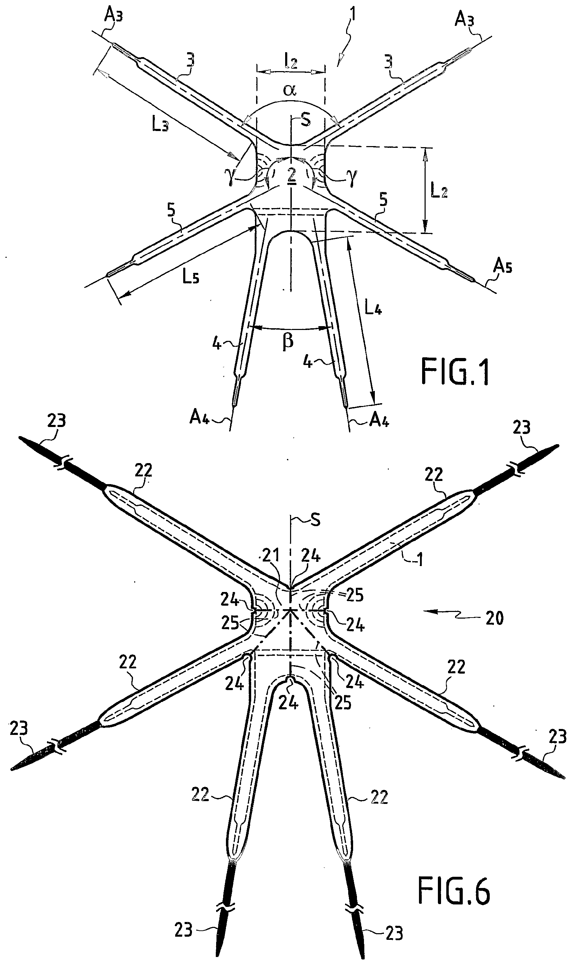

L'invention trouve une application préférée dans le traitement de la cystocèle. A cet effet, l'invention propose un implant plus particulièrement conçu pour cette pathologie et désigné dans son ensemble par la référence **1** à la **fig. 1****.** Cet implant **1** présente une structure mince et souple et se trouve réalisé dans un matériau biocompatible adapté, tel que, par exemple, un matériau synthétique, tissé ou non, ou, encore, tricoté, à base de fibres de polypropylène ou de polyester. Un tel matériau synthétique pourra alors être enduit ou non de produits favorisant la croissance cellulaire. De même, l'implant selon l'invention, pourrait être réalisé en matériaux naturels, tels que du « fascia latta » ou, encore, tout matériel biologique ou synthétique résorbable.

Conformément à une caractéristique essentielle de l'invention, l'implant **1** comprend un corps de support **2** à partir duquel s'étendent deux bretelles de suspension antérieures **3,** disposées de part et d'autre d'un plan sagittal 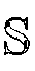 L'implant comprend, également, deux bretelles de suspension postérieures **4,** disposées aussi - de part et d'autre du plan sagittal **S.** L'implant **1** comprend, en outre, deux bretelles de suspension moyennes **5** qui s'étendent à partir du corps de support **2,** de part et d'autre du plan sagittal **S,** entre les bretelles antérieures **3** et postérieures **4** correspondantes.

Selon l'exemple illustré, le corps de support **2** présente une forme sensiblement rectangulaire, sans qu'une telle forme puisse être considérée comme nécessaire au sens de l'invention et les bretelles de suspension antérieures **3** et postérieures **4** s'étendent chacune à partir d'un coin du corps **2.** Il est à noter que, de manière préférée, le plan sagittal **S** correspond à un plan de symétrie de l'implant 1.

Selon une caractéristique, liée à la technique de mise en oeuvre proposée par l'invention, les bretelles antérieures **3** sont destinées à être introduites dans l'un des deux trous ischio-pubien du sujet à traiter. A cet effet, les axes longitudinaux **A₃** des bretelles antérieures **3** forment un angle **α,** supérieur à 45°, de préférence compris entre 100° et 180° en étant, de manière préférée, compris entre 115° et 170°. De plus, afin de permettre une insertion aisée des bretelles antérieures **3** dans les trous ischio-pubiens ou obturés correspondants, ces dernières présentent une longueur **L₃,** mesurée entre l'extrémité distale de chaque bretelle antérieure **3** et le corps de support **2,** supérieure à 100 mm et, de préférence, supérieure ou égale à 120 mm.

Les bretelles postérieures **4** sont, quant à elles, et comme cela apparaîtra par la suite, destinées à passer au travers des ligaments utérosacrés et, à cet effet, les axes longitudinaux **A₄** des bretelles de suspension postérieures **4** forment entre eux un angle β, non nul, de préférence supérieur à 10° et, de manière plus particulièrement préférée, compris entre 10° et 75°. Il est à noter qu'afin de faciliter leur mise en place les bretelles de suspension postérieures **4** présentent une longueur **L₄,** mesurée entre l'extrémité distale des bretelles et le corps de support **2,** de préférence supérieure à 100 mm en étant, de manière plus particulièrement préférée, supérieure ou égale à 120 mm.

Les bretelles de suspension moyennes **5** sont, quant à elles, destinées à être passées au travers des ligaments utérosacrés et, à cet effet, l'axe longitudinal 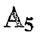 de chaque bretelle de suspension moyenne **5,** forme, avec la partie antérieure du plan sagittal **S,** un angle γ compris entre 100° et 140° et, de préférence, compris entre 110° et 130° et, de manière plus particulièrement préférée, entre 115° et 125°. De même que les autres bretelles de suspension antérieures **3** et postérieures **4,** les bretelles de suspension moyennes **5** présentent une longueur **L5**, mesurée entre l'extrémité distale des bretelles de suspension moyennes **5** et le corps de support **2,** de préférence supérieure à 100 mm et, de manière plus particulièrement préférée, supérieure ou égale à 120 mm.

Par ailleurs, la largeur des bretelles de suspension **3, 4, 5** est choisie pour valoir, de préférence non exclusivement, entre 5 mm et 15 mm et par exemple valoir une dizaine de millimètres.

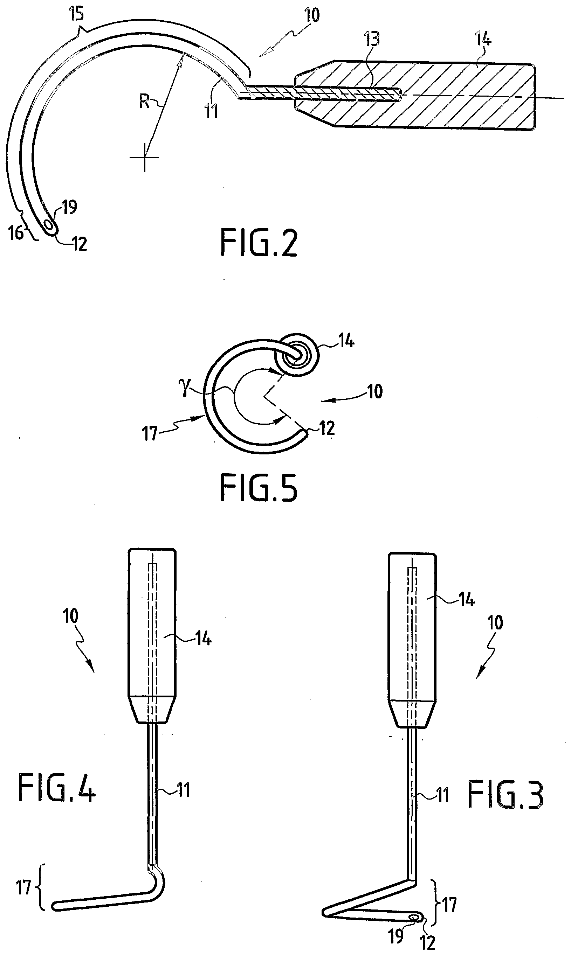

L'implant **1,** tel que décrit précédemment, est destiné à être mis en place au niveau de la paroi vaginale antérieure d'une patiente. A cet effet, afin de réduire au minimum la dissection de cette région et le traumatisme en découlant, l'invention propose au chirurgien procédant au traitement d'utiliser un ou plusieurs guides perforateurs allongés **10,** tels que ceux plus particulièrement illustrés aux **fig. 2** et **3** à **5.**

De manière générale, un tel guide perforateur **10** comprend un corps ou mandrin allongé **11** dont une extrémité **12** est destinée à être introduite dans le corps du sujet à traiter et dont l'autre extrémité **13** est pourvue d'une poignée **14.** Il doit être remarqué que l'extrémité d'introduction **12** est, de préférence, constituée par une pointe mousse, c'est-à-dire une pointe atraumatique qui n'est pas susceptible de blesser ou de couper les tissus dans lesquels elle doit être introduite.

Selon une forme de réalisation illustrée à la **fig. 2****,** le guide perforateur **10** présente une forme arquée dans un plan. Cette forme arquée dans un plan est plus particulièrement adaptée pour la mise en place des bretelles de suspension dans les zones antérieure et postérieure des trous obturés. De manière préférée mais non strictement nécessaire la partie arquée du guide perforateur présente alors un rayon de courbure **R** compris entre 30 mm et 60 mm et, de préférence, pour la partie **15** du guide perforateur **10** s'étendant entre la poignée **14** et l'extrémité **12,** compris entre 40 mm et 50 mm, la partie extrême **16** du guide perforateur **10** présentant alors un rayon de courbure variable.

Selon une autre forme de réalisation du guide perforateur **10,** illustrée aux **fig. 3** à **5****,** le corps allongé **11** du guide **10** présente une extrémité **17** de forme hélicoïdale, également adaptée pour la mise en place des bretelles de suspension dans les zones antérieure ou postérieure des trous obturés. De manière préférée, l'extrémité distale **17** du guide perforateur présente alors la forme d'une portion de spire hélicoïdale s'étendant sur un angle γ compris entre 180° et 360° et, de préférence, compris entre 255° et 270°. De même, de manière préférée, la spire **17** du guide perforateur présente un rayon de courbure compris entre 20 mm et 40 mm, avec un pas compris entre 15 mm et 25 mm.

Le traitement chirurgical de la cystocèle, au moyen d'un implant **1** et de guides perforateurs **10,** tel que décrit précédemment, s'effectue de la manière suivante.

La patiente à traiter fait tout d'abord l'objet d'une anesthésie qui peut être générale ou régionale ou, encore, locale, selon les préférences du chirurgien et l'état de santé de la patiente. La position opératoire de la patiente sur la table d'opération sera celle de la chirurgie vaginale habituelle, à savoir fesses de la patiente légèrement en dehors de la table d'opération et cuisses fléchies modérément sur l'abdomen.

Une dissection de la zone d'intervention est alors effectuée en respectant les attaches du col vésical. On exerce tout d'abord une traction sur le col utérin, au moyen par exemple d'une pince de Muze, afin d'exposer le cul de sac vaginal antérieur. Une incision vaginale, dite précervicale horizontale, peut ainsi être pratiquée sur la face antérieure du col utérin, transversalement sur le versant cervical du cul de sac vaginal, bien exposé par la traction. La tranche de section vaginale antérieure est alors saisie en totalité, peau vaginale et fascia de Halban, au moyen par exemple de trois pinces de Alis qui sont tractées vers le haut pour exposer le plan vésicovaginal. Un décollement vesicovaginal est ensuite conduit progressivement en éversant la paroi vaginale antérieure. Ce décollement est arrêté en bas et au milieu, juste au-dessus du col vésical, tandis qu'il est conduit en bas et latéralement jusqu'à l'*arcatus tendinus fascia pelvis.* Cette zone de l'*arcatus tendinus fascia pelvis* est alors ouverte en direction du foramen obturé, de façon à permettre l'introduction d'un doigt derrière le muscle obturateur. De même, le décollement pratiqué en haut au niveau de l'incision vaginale doit permettre le glissement d'un doigt contre le muscle releveur.

Cette dissection effectuée, il peut être procédé à la mise en place d'une première bretelle de suspension antérieure **3** dans un trou ischio-pubien correspondant. A cet effet, il est utilisé un guide perforateur **10** arqué ou hélicoïdal, tels que décrits précédemment, au choix du chirurgien. Il est, tout d'abord, pratiqué une incision punctiforme située de préférence 15 mm environ en dehors de la branche ischiopubienne au niveau horizontal par rapport au capuchon clitoridien. Un doigt est alors glissé derrière le trou obturé, de manière à recevoir l'extrémité d'introduction ou pointe **12** du guide perforateur **10,** introduite par l'incision punctiforme. Le guide perforateur **10** est alors engagé en étant conduit par le doigt jusqu'à l'incision vaginale antérieure.

A ce stade, la bretelle de suspension antérieure **3** est fixée sur le guide perforateur, par exemple au niveau d'un chas **19** offert par l'extrémité d'introduction **12** de ce guide. Cette fixation réalisée, le guide perforateur est retiré, assurant ainsi une traction sur la bretelle de suspension antérieure qui se trouve alors introduite au travers de foramen obturé.

Le même geste chirurgical est reproduit pour l'introduction de la seconde bretelle de suspension antérieure **3.**

Il est à noter que les bretelles de suspension antérieures **3** sont, de préférence, positionnées sans aucune traction pour prévenir l'effet dysuriant de l'implant **1** qui est disposé juste au-dessus et en arrière du col vésical.

Il est ensuite procédé à la mise en place d'une première bretelle de suspension moyenne **5.**

A cet effet, il est réalisé une incision punctiforme située dans le sillon génito-crural au niveau horizontal par rapport à la fourchette vulvaire. Le guide perforateur **10** est alors introduit par cette incision pour être conduit au travers du muscle releveur. Il doit être remarqué que l'extrémité du guide perforateur **10** est située alors juste en dedans de la branche ischiopubienne, un doigt placé contre la face supérieure du muscle releveur recevant la pointe du guide perforateur pour la conduire jusque dans l'incision vaginale antérieure.

L'extrémité de la bretelle de suspension moyenne **5** à positionner est alors fixée à l'extrémité **12** du guide perforateur **10** qui est ensuite retiré en sens inverse pour entraîner avec lui la bretelle de suspension moyenne **5.**

Un même geste chirurgical est reproduit pour la mise en place de la seconde bretelle de suspension moyenne **5.**

Il doit être remarqué que les bretelles de suspension moyennes **5** sont placées, de préférence, sans aucune traction ou précontrainte, afin de prévenir l'effet de corde de la bretelle prothétique fibrosée dans les gouttières vaginales latéro-vésicales.

Selon une alternative d'implantation, les bretelles de suspension moyennes **5** pourraient également être implantées au travers d'un trou obturé correspondant, dans sa partie postéro-inférieure en regard de l'ischion.

A ce stade de la mise en place de l'implant **1,** ce dernier est, de préférence, fixé au niveau de son bord antérieur par un ou plusieurs et, de préférence, deux ou trois points résorbables à la face antérieure du col utérin.

La mise en place de l'implant **1** se poursuit par l'engagement des bretelles de suspension postérieures **4.** Il doit être remarqué que les bretelles de suspension postérieures **4** sont indispensables dans le cas d'une hystérectomie sans conservation du col.

Une première bretelle de suspension postérieure **4** est placée à travers la base du ligament large et à travers la base du ligament utérosacré. Pour ce faire le guide perforateur **10** est introduit le long de la paroi vaginale d'arrière en avant à travers le ligament utérosacré. La bretelle postérieure **4** est alors fixée au guide perforateur et tractée en sens inverse d'avant en arrière à travers le ligament utérosacré.

Le même geste chirurgical est répété pour la mise en place de la seconde bretelle de suspension postérieure **4.**

Les bretelles de suspension postérieures **4** peuvent alors, soit être laissées libres après la traversée des ligaments utérosacrés, soit, au contraire, être chacune fixée par un point de suture résorbable à la plaque perectale.

Selon une variante d'intervention, les bretelles de suspension postérieures **4** sont chacune passée tout d'abord au travers du ligament utérosacré correspondant, puis au travers du ligament sacrosciatique au moyen du guide perforateur **10** qui aura été passé dans la zone voie transglutéale.

L'opération s'achève par une fermeture de l'incision vaginale au moyen d'un surjet de fil résorbable, ainsi que par la fermeture des incisions punctiformes au moyen d'un surjet de fil résorbable également.

En fin d'intervention, il est mis en place une mèche vaginale, ainsi qu'une sonde vésicale qui seront retirées quarante-huit heures après l'intervention. Les résidus post-mictionnels étant alors mesurés par sondage, afin de s'assurer que la vidange vésicale est satisfaisante, de manière à autoriser une sortie de la patiente.

L'intervention pour le traitement de la cystocèle aura une durée d'une heure environ et il convient d'adopter une durée d'hospitalisation moyenne de quatre jours. L'activité de la patiente sera limitée pendant un mois et il conviendra d'éviter tout bain pendant cette même période. Enfin, il conviendra de prévoir une période d'abstinence sexuelle de six semaines après l'opération.

La technique proposée permet ainsi de ne traiter que la pathologie, à savoir le déséquilibre de la statique pelvienne et donc de restituer une anatomie la plus normale possible en conservant le schéma corporel de l'individu. Cette technique permet, de manière avantageuse, de conserver les organes sains ou n'influençant pas défavorablement la statique pelvienne. En effet, la pathologie cancéreuse aura été écartée par le bilan pré-opératoire et il sera possible d'assurer après l'intervention chirurgicale une surveillance gynécologique fiable.

Par ailleurs, il existe de très faibles risques de cancer génital pelvien et, de plus, le traitement, proposé par l'invention, ne complique pas un accès ultérieur aux organes génitaux.

Comme cela a été indiqué précédemment, l'implant selon l'invention est, de préférence, disposé de manière à ne présenter aucune tension résiduelle après sa mise en place. Afin de faciliter ce geste opératoire, l'invention propose dans une variante d'intervention de mettre en oeuvre un introducteur, plus particulièrement illustré à la **fig. 6** et désigné dans son ensemble par la référence **20.**

Cet introducteur présente une structure souple et de forme analogue à celle de l'implant. L'introducteur **20** est, de préférence, réalisé dans un matériau polymère biocompatible de la famille des matières plastiques à faible coefficient de friction, tel que, par exemple, le polyéthylène. L'introducteur **20** comprend alors un corps creux **21** définissant une cavité de réception du corps **2** de l'implant **1.** L'introducteur **20** comprend, également, des branches tubulaires **22** qui s'étendent à partir du corps creux **21** et qui définissent chacune une cavité de réception d'une bretelle de suspension **3, 4** et **5** de l'implant **1.** Chaque branche tubulaire **22** présente alors des moyens de traction **23** s'étendant à partir de l'extrémité libre de la branche **22** correspondante. Les moyens de traction **23** peuvent être réalisés de toute façon appropriée comme, par exemple, par des systèmes d'accrochage des extrémités des branches **22** sur un guide perforateur **10.** Selon l'exemple illustré à la **fig. 6****,** les moyens de traction **23** comprennent, pour chaque branche **22,** une aiguille souple ou semi-rigide avec une extrémité atraumatique ou mousse. Une telle aiguille peut être réalisée dans le même matériau que le matériau constitutif de l'introducteur **20** ou, plus généralement, en matière plastique biocompatible présentant, de préférence, un faible coefficient de friction.

L'introducteur **20** comprend, enfin, des moyens de découpe **24,** dont la fonction apparaîtra par la suite, du corps creux **21** au moins de l'introducteur **20.** Les moyens de découpe **24** peuvent être alors réalisés de toute façon appropriée et, selon l'exemple illustré, comprennent une série de six ouvertures **24** réalisées à la périphérie du corps creux **21,** entre chacune des branches tubulaires **22,** pour permettre le passage d'un outil de coupe pour assurer une découpe du corps creux **21** selon des lignes **25** matérialisées par des traits mixtes à la **fig. 6****.**

L'implant **1** se trouve disposé à l'intérieur du corps creux **21** et des branches tubulaires **22** en étant, de préférence, libre à l'intérieur de ces derniers, de manière que les efforts exercés sur l'introducteur **20** ne soient pas transmis à l'implant **1** en lui-même.

La mise en place de l'implant **1,** au moyen de l'introducteur **20** ainsi constitué, s'effectue selon la même procédure opératoire que décrite précédemment, le retrait et la découpe de l'introducteur **20** intervenant après la mise en place dans le corps de la patiente de l'ensemble des branches de suspension ou, plus exactement, des branches tubulaires correspondantes **22.** Le retrait des différents éléments constitutifs de l'introducteur **20** par des tractions exercées deux à deux sur les branches tubulaires **22** opposées permet ainsi de déposer l'implant **1,** sans aucune contrainte, sur ce dernier, de sorte qu'il se trouve dans un état qui pourrait être qualifié de détendu.

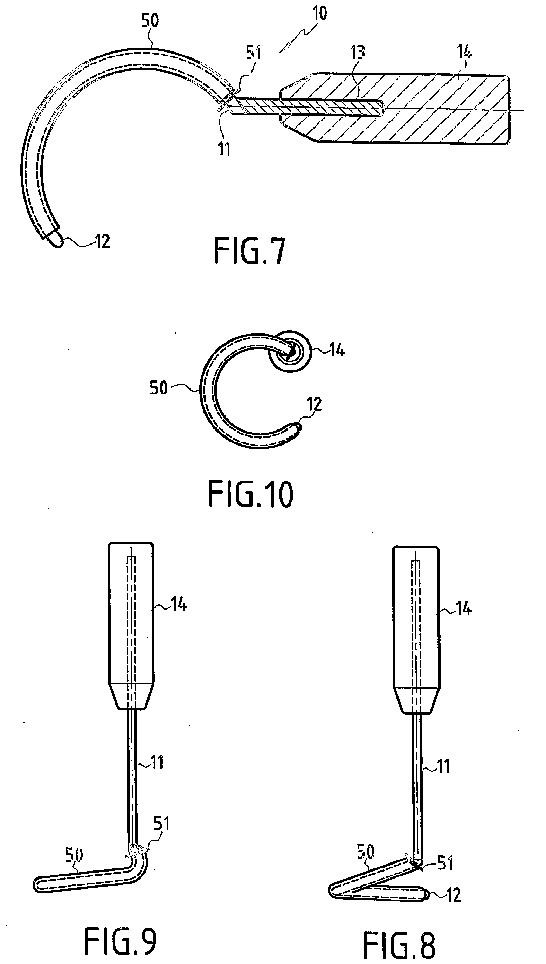

Dans les exemples de traitement et d'opération décrits précédemment, le dispositif d'introduction utilisé comprend de simples guides perforateurs **10.** Toutefois, afin de tenter de réduire au maximum le traumatisme par abrasion des zones tissulaires traversées, il peut être envisagé de mettre en oeuvre un ancillaire associant le guide perforateur **10** à une chemise souple **50** de forme complémentaire à celle du guide **10,** comme cela est illustré aux **fig. 7** et **8** à **10.** La chemise **50** est engagée sur le guide perforateur **10** qui présente alors une butée ou garde **51** sur laquelle la chemise **50** vient en appui lors de l'introduction du guide perforateur **10** dans le corps de la patiente. La chemise **50** est laissée en place dans le corps de la patiente après retrait du guide perforateur **10** et avant la mise place de l'implant **1, 29, 39.** La chemise utilisée permet ainsi de créer un canal pour le passage d'un élément de traction **23** de l'introducteur **20** et dans lequel une branche tubulaire **23** et la bretelle de suspension **3, 4, 5** associée ou une bretelle de suspension **3, 4, 5** peut être déplacée par glissement, de manière à régler la position de l'implant **1, 29, 39** sans abrasion des tissus traversés. Il est alors utilisé une chemise **50** pour la mise en place de chaque bretelle de suspension **3, 4, 5.** Les chemises **50** sont ensuite retirées en même temps que les branches tubulaires **23** enveloppant les branches de suspension de l'implant ou lorsque l'implant est posé nu après réglage de la position de l'implant.

Ainsi, la mise en oeuvre des chemises **50** évite les phénomènes inflammatoires aigus et réduit le traumatisme tissulaire, dans la mesure où les sites d'implantation sont composés de tissus musculaires très spécialisés qui ont perdu une grande partie de leurs capacités de régénération et cicatrisation rapide.

Bien entendu, diverses autres modifications peuvent être apportées à l'invention sans sortir de son cadre.

## Revendications

1. Implant, pour le traitement de la cystocèle, présentant une structure mince et souple, **caractérisé en ce qu'**il comprend un corps de support **(2)** à partir duquel s'étendent au moins :
▪ deux bretelles de suspension antérieures **(3),** disposées de part et d'autre d'un plan sagittal **(S)** et destinées à être introduites dans un trou ischio-pubien, les axes longitudinaux **(A₃)** des bretelles antérieures **(3)** formant, entre eux, un angle (α) supérieur à 45°,
▪ deux bretelles de suspension postérieures **(4),** disposées de part et d'autre du plan sagittal **(S)** et destinées à passer au travers de ligaments utéro sacrés, les axes longitudinaux **(A₄)** des bretelles postérieures **(4)** formant entre eux un angle **(β)** non nul,
▪ et deux bretelles de suspension moyennes **(5)** disposées de part et d'autre du plan sagittal **(S)** et entre les bretelles antérieures **(3)** et postérieures **(4)** et destinées à passer au travers de ligaments utéro sacrés, l'axe longitudinal **(A₅)** de chaque bretelle de suspension moyenne **(5),** formant, avec la partie antérieure du plan sagittal **(S),** un angle (γ) compris entre 100° et 140° et, de préférence, compris entre 110° et 130°.

2. Implant selon la revendication 1, **caractérisé en ce que** les axes longitudinaux **(A₃)** des bretelles antérieures **(3)** forment un angle (α) compris entre 100° et 180°.

3. Implant selon la revendication 1, **caractérisé en ce que** l'angle (α) est compris entre 115° et 170°.

4. Implant selon la revendication 1, **caractérisé en ce que** l'angle (β) est supérieur à 10°.

5. Implant selon la revendication 4, **caractérisé en ce que** l'angle (β) est compris entre 10 et 75°.*

6. Implant selon la revendication 5, **caractérisé en ce que** l'angle (β) est compris entre 100° et 180°.

7. Implant selon l'une des revendications 1 à 6, **caractérisé en ce que** les bretelles antérieures **(3)** présentent une longueur supérieure à 100 mm et, de préférence, supérieure à 120 mm.

8. Implant selon l'une des revendications 1 à 7, **caractérisé en ce que** les bretelles postérieures **(4)** présentent une longueur supérieure à 100 mm et, de préférence, supérieure ou égale à 120 mm.

9. Implant selon l'une des revendications 1 à 8, **caractérisé en ce que** les bretelles moyennes **(5)** présentent une longueur supérieure à 100 mm et, de préférence, supérieure ou égale à 120 mm.

10. Implant selon l'une des revendications 1 à 8, **caractérisé en ce que** le corps de support **(2)** présente une forme générale sensiblement rectangulaire.

11. Implant selon la revendication 10, **caractérisé en ce que** le corps de support **(2)** présente une longueur **(L₂)** comprise entre 60 et 90 mm et une largeur **(I₂)** comprise entre 40 et 60 mm.

12. Implant selon la revendication 10 ou 11, **caractérisé en ce que** les bretelles antérieures **(3)** s'étendent sensiblement à partir des coins antérieurs du corps de support **(2).**

13. Implant selon l'une des revendications 10 à 12, **caractérisé en ce que** les bretelles postérieures **(4)** s'étendent sensiblement à partir des coins postérieurs du corps de support **(2).**

14. Dispositif pour l'introduction d'un implant **(1)** selon l'une des revendications 1 à 13, **caractérisé en ce qu'**il comprend un introducteur **(20)** qui présente une structure souple et une forme analogue à celle de l'implant **(1)** et qui comprend :
▪ un corps creux **(21)** définissant une cavité de réception du corps **(2)** de l'implant **(1),**
▪ des branches tubulaires **(22)** s'étendant à partir du corps creux **(21)** et définissant chacune une cavité de réception d'une bretelle de suspension **(3, 4, 5)** de l'implant **(1),**
▪ des moyens de traction **(23)** s'étendant à partir de l'extrémité de chacune des branches **(22)** du dispositif introducteur,
▪ et des moyens **(25)** de découpe du corps creux **(21)** au moins de l'introducteur **(20).**

15. Dispositif d'introduction selon la revendication 14, **caractérisé en ce que** les moyens de traction **(23)** comprennent, pour chaque branche tubulaire **(21),** une aiguille semi-rigide.

16. Dispositif d'introduction selon la revendication 14 ou 15, **caractérisé en ce que** les moyens de découpe comprennent au moins une ouverture **(24)** pour le passage d'un instrument de coupe.

17. Dispositif d'introduction selon l'une des revendications 14 à 16, **caractérisé en ce qu'**il comprend un implant **(1)** selon l'une des revendications 1 à 16 disposé dans la cavité du corps creux **(21)** et des branches tubulaires **(22).**

18. Dispositif d'introduction selon la revendication 17, **caractérisé en ce que** l'implant **(1)** est libre à l'intérieur de l'introducteur **(10).**

19. Dispositif d'introduction selon des revendications 14 à 18, **caractérisé en ce qu'**il comprend, en outre, un guide perforateur allongé **(10)** ou trocart dont une extrémité **(12)** est destinée à être introduite dans le corps de la patiente et dont l'autre extrémité est pourvue d'une poignée **(14).**

20. Dispositif d'introduction selon la revendication 19, **caractérisé en ce que** le guide perforateur **(10)** présente une forme arquée dans un plan.

21. Dispositif d'introduction selon la revendication 20, **caractérisé en ce que** la partie arquée **(15)** du perforateur **(10)** s'étend sur un secteur angulaire supérieur à 140° et, de préférence, inférieur à 180° et, de manière particulièrement préférée, compris entre 150° et 170°.

22. Dispositif d'introduction selon la revendication 20 ou 21, **caractérisé en ce que** la partie arquée **(15)** du guide perforateur **(10)** présente alors un rayon de courbure (R) compris entre 30 mm et 60 mm et, de préférence, pour la partie du guide perforateur s'étendant entre la poignée et l'extrémité destinée à être introduite dans le corps du patient, compris entre 40 mm et 50 mm.

23. Dispositif d'introduction selon la revendication 19, **caractérisé en ce que** le guide perforateur **(10)** présente, au niveau de son extrémité opposée à la poignée ou distale **(17),** une forme hélicoïdale.

24. Dispositif d'introduction selon la revendication 23, **caractérisé en ce que** l'extrémité distale **(17)** du guide perforateur **(10)** présente la forme d'une portion de spire hélicoïdale s'étendant sur un angle compris entre 180° et 350° et, de préférence, compris entre 255° et 270°.

25. Dispositif d'introduction selon la revendication 24, **caractérisé en ce que** la spire **(17)** du guide perforateur **(10)** présente un rayon de courbure compris entre 20 mm et 40 mm, avec un pas compris entre 15 mm et 25 mm.

26. Dispositif d'introduction selon l'une des revendications 19 à 25, **caractérisé en ce qu'**il comprend, en outre, une chemise tubulaire amovible **(50)** de forme complémentaire à celle du guide perforateur **(10),** destinée à être engagée sur le guide perforateur **(10)** et à rester dans le corps de la patiente après retrait du guide perforateur **(10)** pour définir un tunnel pour le passage des moyens de traction **(23)** de l'introducteur **(20).**

## Patentansprüche

1. Implantat zur Behandlung einer Zystozele, aufweisend eine dünne und weiche Struktur, **dadurch gekennzeichnet, dass** es einen Tragekörper (2) umfasst, von dem sich mindestens erstrecken:
▪ zwei anteriore Aufhängungsträger (3), die auf beiden Seiten einer Sagittalebene (S) angeordnet sind und dazu vorgesehen sind, in ein ischiopubisches Loch eingeführt zu werden, wobei die Längsachsen (A₃) der anterioren Träger (3) untereinander einen Winkel (α) bilden, der größer als 45° ist,
▪ zwei posteriore Aufhängungsträger (4), die auf beiden Seiten der Sagittalebene (S) angeordnet sind und dazu vorgesehen sind, durch Uterosakralligamente hindurchzutreten, wobei die Längsachsen (A₄) der posterioren Träger (4) untereinander einen Winkel (β) bilden, der nicht null ist,
▪ und zwei mittlere Aufhängungsträger (5), die auf beiden Seiten der Sagittalebene (S) und zwischen den anterioren (3) und den posterioren (4) Trägern angeordnet sind und dazu vorgesehen sind, durch Uterosakralligamente hindurchzutreten, wobei die Längsachse (A₅) jedes mittleren Aufhängungsträgers (5) mit dem anterioren Teil der Sagittalebene (S) einen Winkel (γ) bildet, der zwischen 100° und 140° liegt und vorzugsweise zwischen 110° und 130° liegt.

2. Implantat nach Anspruch 1, **dadurch gekennzeichnet, dass** die Längsachsen (A₃) der anterioren Träger (3) einen Winkel (α) bilden, der zwischen 100° und 180° liegt.

3. Implantat nach Anspruch 1, **dadurch gekennzeichnet, dass** der Winkel (α) zwischen 115° und 170° liegt.

4. Implantat nach Anspruch 1, **dadurch gekennzeichnet, dass** der Winkel (β) größer als 10° ist.

5. Implantat nach Anspruch 4, **dadurch gekennzeichnet, dass** der Winkel (β) zwischen 10 und 75° liegt.

6. Implantat nach Anspruch 5, **dadurch gekennzeichnet, dass** der Winkel (β) zwischen 100° und 180° liegt.

7. Implantat nach einem der Ansprüche 1 bis 6, **dadurch gekennzeichnet, dass** die anterioren Träger (3) eine Länge aufweisen, die größer als 100 mm und vorzugsweise größer als 120 mm ist.

8. Implantat nach einem der Ansprüche 1 bis 7, **dadurch gekennzeichnet, dass** die posterioren Träger (4) eine Länge aufweisen, die größer als 100 mm und vorzugsweise größer als oder gleich 120 mm ist.

9. Implantat nach einem der Ansprüche 1 bis 8, **dadurch gekennzeichnet, dass** die mittleren Träger (5) eine Länge aufweisen, die größer als 100 mm und vorzugsweise größer als oder gleich 120 mm ist.

10. Implantat nach einem der Ansprüche 1 bis 8, **dadurch gekennzeichnet, dass** der Tragekörper (2) eine im Wesentlichen rechteckige allgemeine Form aufweist.

11. Implantat nach Anspruch 10, **dadurch gekennzeichnet, dass** der Tragekörper (2) eine Länge (L₂), die zwischen 60 und 90 mm liegt, und eine Breite (l₂), die zwischen 40 und 60 mm liegt, aufweist.

12. Implantat nach Anspruch 10 oder 11, **dadurch gekennzeichnet, dass** die anterioren Träger (3) sich im Wesentlichen von anterioren Ecken des Tragekörpers (2) erstrecken.

13. Implantat nach einem der Ansprüche 10 bis 12, **dadurch gekennzeichnet, dass** die posterioren Träger (4) sich im Wesentlichen von posterioren Ecken des Tragekörpers (2) erstrecken.

14. Vorrichtung zum Einführen eines Implantats (1) nach einem der Ansprüche 1 bis 13, **dadurch gekennzeichnet, dass** sie einen Einführer (20) umfasst, der eine weiche Struktur und eine zu der des Implantats (1) analoge Form aufweist und der umfasst:
▪ einen hohlen Körper (21), der einen Hohlraum zur Aufnahme des Körpers (2) des Implantats (1) definiert,
▪ röhrenförmige Arme (22), die sich von dem hohlen Körper (21) erstrecken und jeweils einen Hohlraum zur Aufnahme eines Aufhängungsträgers (3, 4, 5) des Implantats (1) definieren,
▪ Zugmittel (23), die sich von dem Ende jedes der Arme (22) der Einführervorrichtung erstrecken,
▪ und Mittel (25) zum Schneiden mindestens des Einführers (20) des hohlen Körpers (21).

15. Einführungsvorrichtung nach Anspruch 14, **dadurch gekennzeichnet, dass** die Zugmittel (23) für jeden röhrenförmigen Arm (21) eine halbstarre Nadel umfassen.

16. Einführungsvorrichtung nach Anspruch 14 oder 15, **dadurch gekennzeichnet, dass** die Schneidmittel mindestens eine Öffnung (24) zum Hindurchtreten eines Schneidinstruments umfassen.

17. Einführungsvorrichtung nach einem der Ansprüche 14 bis 16, **dadurch gekennzeichnet, dass** sie ein Implantat (1) nach einem der Ansprüche 1 bis 16 umfasst, das in dem Hohlraum des hohlen Körpers (21) und den röhrenförmigen Armen (22) angeordnet ist.

18. Einführungsvorrichtung nach Anspruch 17, **dadurch gekennzeichnet, dass** das Implantat (1) im Inneren des Einführers (10) frei ist.

19. Einführungsvorrichtung nach einem der Ansprüche 14 bis 18, **dadurch gekennzeichnet, dass** sie außerdem eine längliche Perforatorführung (10) oder einen Trokar umfasst, deren bzw. dessen Ende (12) dazu vorgesehen ist, in den Körper der Patientin eingeführt zu werden und deren bzw. dessen anderes Ende mit einem Griff (14) versehen ist.

20. Einführungsvorrichtung nach Anspruch 19, **dadurch gekennzeichnet, dass** die Perforatorführung (10) in einer Ebene eine gebogene Form aufweist.

21. Einführungsvorrichtung nach Anspruch 20, **dadurch gekennzeichnet, dass** der gebogene Teil (15) des Perforators (10) sich auf einem winkelförmigen Sektor, der größer als 140° und vorzugsweise kleiner als 180° ist und besonders bevorzugt zwischen 150° und 170° liegt, erstreckt.

22. Einführungsvorrichtung nach Anspruch 20 oder 21, **dadurch gekennzeichnet, dass** der gebogene Teil (15) der Perforatorführung (10) dann einen Krümmungsradius (R) aufweist, der zwischen 30 mm und 60 mm liegt und vorzugsweise für den Teil der Perforatorführung, der sich zwischen dem Griff und dem Ende erstreckt, das dazu vorgesehen ist, in den Körper des Patienten eingeführt zu werden, zwischen 40 mm und 50 mm liegt.

23. Einführungsvorrichtung nach Anspruch 19, **dadurch gekennzeichnet, dass** die Perforatorführung (10) auf Höhe ihres zu dem Griff entgegengesetzten oder distalen (17) Endes eine Spiralform aufweist.

24. Einführungsvorrichtung nach Anspruch 23, **dadurch gekennzeichnet, dass** das distale Ende (17) der Perforatorführung (10) die Form eines Abschnitts einer spiralförmigen Windung aufweist, die sich in einem Winkel erstreckt, der zwischen 180° und 350° liegt und vorzugsweise zwischen 255° und 270° liegt.

25. Einführungsvorrichtung nach Anspruch 24, **dadurch gekennzeichnet, dass** die Windung (17) der Perforatorführung (10) einen Krümmungsradius, der zwischen 20 mm und 40 mm liegt, mit einem Gang, der zwischen 15 mm und 25 mm liegt, aufweist.

26. Einführungsvorrichtung nach einem der Ansprüche 19 bis 25, **dadurch gekennzeichnet, dass** sie außerdem eine abnehmbare röhrenförmige Hülle (50) in einer zu der Perforatorführung (10) komplementären Form umfasst, die dazu vorgesehen ist, auf der Perforatorführung (10) in Eingriff gebracht zu werden und nach Zurückziehen der Perforatorführung (10) in dem Körper der Patientin zu verbleiben, um einen Tunnel zum Hindurchtreten von Zugmitteln (23) des Einführers (20) zu definieren.

## Claims

1. An implant, for the treatment of a cystocele, having a thin and flexible structure, **characterized in that** it comprises a support body (2) from which extend at least:
▪ two anterior suspension straps (3), disposed on either side of a sagittal plane (S) and designed to be introduced into an ischiopubic hole, the longitudinal axes (A₃) of the anterior straps (3) forming between them an angle (α) greater than 45°,
▪ two posterior suspension straps (4), disposed on either side of the sagittal plane (S) and designed to pass through utero sacral ligaments, the longitudinal axes (A₄) of the posterior straps (4) forming between them a non-zero angle (β),
▪ and two median suspension straps (5) disposed on either side of the sagittal plane (S) and between the anterior (3) and posterior (4) straps and designed to pass through utero sacral ligaments, the longitudinal axis (A₅) of each median suspension strap (5), forming, with the anterior portion of the sagittal plane (S), an angle (γ) comprised between 100° and 140° and preferably comprised between 110° and 130°.

2. The implant according to claim 1, **characterized in that** the longitudinal axes (A₃) of the anterior straps (3) form an angle (α) comprised between 100° and 180°.

3. The implant according to claim 1, **characterized in that** the angle (α) is comprised between 115° and 170°.

4. The implant according to claim 1, **characterized in that** the angle (β) is greater than 10°.

5. The implant according to claim 4, **characterized in that** the angle (β) is comprised between 10 and 75°.

6. The implant according to claim 5, **characterized in that** the angle (β) is comprised between 100° and 180°.

7. The implant according to one of claims 1 to 6, **characterized in that** the anterior straps (3) have a length greater than 100 mm and, preferably, greater than 120 mm.

8. The implant according to one of claims 1 to 7, **characterized in that** the posterior straps (4) have a length greater than 100 mm and, preferably, greater than or equal to 120 mm.

9. The implant according to one of claims 1 to 8, **characterized in that** the median straps (5) have a length greater than 100 mm and, preferably, greater than or equal to 120 mm.

10. The implant according to one of claims 1 to 8, **characterized in that** the support body (2) has a substantially rectangular shape.

11. The implant according to claim 10, **characterized in that** the support body (2) has a length (L₂) comprises between 60 and 90 mm and a width (l₂) comprised between 40 and 60 mm.

12. The implant according to claim 10 or 11, **characterized in that** the anterior straps (3) extend substantially from the anterior corners of the support body (2) .

13. The implant according to one of claims 10 to 12, **characterized in that** the posterior straps (4) extend substantially from the posterior corners of the support body (2).

14. A device for the introduction of an implant (1) according to one of claims 1 to 13, **characterized in that** it comprises an introducer (20) which has a flexible structure and a shape similar to that of the implant (1) and which comprises:
▪ a hollow body (21) defining a cavity for receiving the body (2) of the implant (1),
▪ tubular branches (22) extending from the hollow body (21) and each defining a cavity for receiving a suspension strap (3, 4, 5) of the implant (1),
▪ traction means (23) extending from the end of each of the branches (22) of the introduction device,
▪ and means (25) for cutting of the hollow body (21) at least of the introducer (20).

15. An introduction device according to claim 14, **characterized in that** the traction means (23) comprise, for each tubular branch (21), a semi-rigid pin.

16. The introduction device according to claim 14 or 15, **characterized in that** the cutting means comprise at least one opening (24) for the passage of a cutting instrument.

17. The introduction device according to one of claims 14 to 16, **characterized in that** it comprises an implant (1) according to one of claims 1 to 16 disposed in the cavity of the hollow body (21) and of the tubular branches (22).

18. The introduction device according to claim 17, **characterized in that** the implant (1) is free in the interior of the introducer (10).

19. The introduction device according to claims 14 to 18, **characterized in that** it also comprises an elongated perforator guide (10) or trocar of which one end (12) is designed to be introduced into the body of the patient and of which the other end is provided with a handle (14).

20. The introduction device according to claim 19, **characterized in that** the perforator guide (10) has a shape that is arched in a plane.

21. The introduction device according to claim 20, **characterized in that** the arched portion (15) of the perforator (10) extends over an angular sector greater than 140° and, preferably, less than 180° and, in a particularly preferred manner, comprised between 150° and 170°.

22. The introduction device according to claim 20 or 21, **characterized in that** the arched portion (15) of the perforator guide (10) then has a radius of curvature (R) comprised between 30 mm and 60 mm and, preferably, for the portion of the perforator guide extending between the handle and the end designed to be introduced into the body of the patient, comprised between 40 mm and 50 mm.

23. The introduction device according to claim 19, **characterized in that** the perforator guide (10) has, at its end opposite to the handle, or distal (17), a helical shape.

24. The introduction device according to claim 23, **characterized in that** the distal end (17) of the perforator guide (10) has the shape of a portion of a helical coil extending over an angle comprised between 180° and 350° and, preferably, comprised between 255° and 270°.

25. The introduction device according to claim 24, **characterized in that** the coil (17) of the perforator guide (10) has a radius of curvature comprised between 20 mm and 40 mm, with a pitch comprised between 15 mm and 25 mm.

26. The introduction device according to one of claims 19 to 25, **characterized in that** it comprises, in addition, a tubular removable sheath (50) with a shape complementary to that of the perforator guide (10), designed to be engaged on the perforator guide (10) and to remain in the body of the patient after removal of the perforator guide (10) for defining a tunnel for the passage of the traction means (23) of the introducer (20).
